# EUROPEAN PATENT APPLICATION

(11) **EP 4 309 733 A1**
(43) Date of publication of application: **24.01.2024**
(21) Application number: 22306096.3
(22) Date of filing: 22.07.2022
(51) Int. Cl.: A61P 9/00, C07K 16/18

(54) **NEUTRALIZATION OF ACYL-COA BINDING PROTEIN FOR THE TREATMENT OF CARDIAC DYSFUNCTION**

(71) Applicant: Institut National de la Santé et de la Recherche Médicale (INSERM), 75013 Paris (FR); Université Paris Cité, 75006 Paris (FR); Sorbonne Université, 75006 Paris (FR); Assistance Publique-Hôpitaux de Paris (APHP), 75004 Paris (FR)
(72) Inventor: The designation of the inventor has not yet been filed
(74) Representative: Inserm Transfert

(57) **Abstract**

Cardiomyopathies are heart muscle disorders which represent a heterogeneous group of diseases that often lead to progressive heart failure with significant morbidity and mortality. Here, the inventors show that neutralization of ACBP/DBI reduces the deleterious effect of the anthracycline doxorubicin on cardiac function. Since anthracycline-induced heart failure is a model of accelerated cardiac aging, it is also plausible to use ACBP/DBI inhibition or neutralization as a method to prevent or treat aging of the cardiovascular system. The inventors also found that genetic and pharmacological interventions to deplete ACBP efficiently improve cardiac dysfunction associated with experimental HFpEF, a condition associated with aging, hypertension and obesity. Accordingly, the present invention relates to methods for the treatment of cardiac dysfunction comprising neutralization of Acyl-CoA Binding Protein.

## Description

### FIELD OF THE INVENTION:

The present invention is in the field of medicine, in particular cardiology.

### BACKGROUND OF THE INVENTION:

Cardiomyopathies are heart muscle disorders which represent a heterogeneous group of diseases that often lead to progressive heart failure with significant morbidity and mortality. Common symptoms include dyspnea and peripheral oedema, and risks of having dangerous forms of irregular heart rate and sudden cardiac death are increased. For instance, heart failure with preserved ejection fraction (HFpEF) is currently the predominant form of heart failure and the leading cause of hospitalization in the elderly. However, limited understanding of the underlying mechanisms of HFpEF has led to a longstanding absence of evidence-based therapies. In this respect, growing epidemiological and experimental evidence suggests that metabolic dysfunction might drive the pathogenesis of HFpEF. In fact, the majority of HFpEF patients are obese or diabetic, suggesting that metabolic therapies acting on the heart and peripheral organs are worth considering *(*Sedej S, Abdellatif M. Metabolic therapy for managing heart failure with preserved ejection fraction. J Mol Cell Cardiol 2022;168:68-9 doi 10.1016/j.yjmcc.2022. 04. 009*).*

Acyl coenzyme A binding protein (ACBP), which is encoded by *diazepam-binding inhibitor* (*DBI*), is a leaderless polypeptide that can be secreted in an unconventional fashion during the activation of autophagy *(*Bravo-San Pedro JM, Sica V, Martins I, Pol J, Loos F, Maiuri MC, et al. Acyl-CoA-Binding Protein Is a Lipogenic Factor that Triggers Food Intake and Obesity. Cell Metab 2019;30(4):754-67 e9 doi 10.1016/j.cmet.2019.07.010*).* Recently, it was found that circulating ACBP/DBI levels increase with aging and obesity *(*Bravo-San Pedro JM, Sica V, Martins I, Pol J, Loos F, Maiuri MC, et al. Acyl-CoA-Binding Protein Is a Lipogenic Factor that Triggers Food Intake and Obesity. Cell Metab 2019;30(4):754-67 e9 doi 10.1016/j.cmet.2019.07.010*;* Joseph A, Chen H, Anagnostopoulos G, Montegut L, Lafarge A, Motino O, et al. Effects of acyl-coenzyme A bindingprotein (ACBP)/diazepam-binding inhibitor (DBI) on body mass index. Cell Death Dis 2021;12(6):599 doi 10.1038/s41419-021-03864-9*).* However, the role of ACBP in cardiomyopathies has never been investigated.

### SUMMARY OF THE INVENTION:

The present invention is defined by the claims. In particular, the present invention relates to methods for the treatment of cardiac dysfunction comprising neutralization of Acyl-CoA Binding Protein.

### DETAILED DESCRIPTION OF THE INVENTION:

The first object of the present invention relates to a method of treating cardiac dysfunction in patient in need thereof comprising administering a therapeutically effective amount of an agent that inhibits the activity or expression of DBI.

As used herein, the term **"cardiac dysfunction"** also referred to as **"myocardial dysfunction"** is known by the person skilled in the art. The term relates to any kind of heart dysfunction, more particularly, the term relates to heart dysfunction affecting the pumping capability of the heart. In particular, the term **"cardiac dysfunction"** relates to a condition in which myocardial contractility, metabolism and ventricular function are reduced in order to cope with a reduced oxygen supply. Typically, cardiac dysfunction involves cardiac remodeling and/or cardiac fibrosis and/or impairment of systolic function (left ventricular ejection fraction (LVEF) function or strain) and/or impairment of diastolic dysfunction. Cardiac dysfunction may be asymptomatic and can occur out of any heart failure symptoms.

As used herein, the term **"treatment"** or "treat" refer to both prophylactic or preventive treatment as well as curative or disease modifying treatment, including treatment of patient at risk of contracting the disease or suspected to have contracted the disease as well as patients who are ill or have been diagnosed as suffering from a disease or medical condition, and includes suppression of clinical relapse. The treatment may be administered to a patient having a medical disorder or who ultimately may acquire the disorder, in order to prevent, cure, delay the onset of, reduce the severity of, or ameliorate one or more symptoms of a disorder or recurring disorder, or in order to prolong the survival of a patient beyond that expected in the absence of such treatment. By **"therapeutic regimen"** is meant the pattern of treatment of an illness, e.g., the pattern of dosing used during therapy. A therapeutic regimen may include an induction regimen and a maintenance regimen. The phrase **"induction regimen"** or **"induction period"** refers to a therapeutic regimen (or the portion of a therapeutic regimen) that is used for the initial treatment of a disease. The general goal of an induction regimen is to provide a high level of drug to a patient during the initial period of a treatment regimen. An induction regimen may employ (in part or in whole) a **"loading regimen",** which may include administering a greater dose of the drug than a physician would employ during a maintenance regimen, administering a drug more frequently than a physician would administer the drug during a maintenance regimen, or both. The phrase **"maintenance regimen"** or **"maintenance period"** refers to a therapeutic regimen (or the portion of a therapeutic regimen) that is used for the maintenance of a patient during treatment of an illness, e.g., to keep the patient in remission for long periods of time (months or years). A maintenance regimen may employ continuous therapy (e.g., administering a drug at a regular interval, e.g., weekly, monthly, yearly, etc.) or intermittent therapy (e.g., interrupted treatment, intermittent treatment, treatment at relapse, or treatment upon achievement of a particular predetermined criteria [e.g., pain, disease manifestation, etc.]).

In particular, the therapeutic method of the present invention is thus particularly suitable for the treatment of cardiac dysfunction in elderly patients, and/or obese patients and/or patients who exhibit one or more risk factors for cardiac dysfunction (e.g. age, alcohol consumption, cigarette smoking, metabolic syndrome, obesity, diabetes/insulin resistance, hypertension, dyslipidaemia, live disease or chronic kidney disease).

As used herein, the term **"elderly patient"** refers to an adult patient sixty-five years of age or older.

As used herein, the term **"obesity"** refers to a condition characterized by an excess of body fat. The operational definition of obesity is based on the Body Mass Index (BMI), which is calculated as body weight per height in meter squared (kg/m²). Obesity refers to a condition whereby an otherwise healthy subject has a BMI greater than or equal to 30 kg/m², or a condition whereby a subject with at least one co-morbidity has a BMI greater than or equal to 27 kg/m². An **"obese subject"** is an otherwise healthy subject with a BMI greater than or equal to 30 kg/m² or a subject with at least one co-morbidity with a BMI greater than or equal 27 kg/m². A **"subject at risk of obesity"** is an otherwise healthy subject with a BMI of 25 kg/m² to less than 30 kg/m² or a subject with at least one co-morbidity with a BMI of 25 kg/m² to less than 27 kg/m². The increased risks associated with obesity may occur at a lower BMI in people of Asian descent. In Asian and Asian-Pacific countries, including Japan, **"obesity"** refers to a condition whereby a subject has a BMI greater than or equal to 25 kg/m². An "obese subject" in these countries refers to a subject with at least one obesity-induced or obesity-related co-morbidity that requires weight reduction or that would be improved by weight reduction, with a BMI greater than or equal to 25 kg/m². In these countries, a "subject at risk of obesity" is a person with a BMI of greater than 23 kg/m2 to less than 25 kg/m².

In some embodiments, the patient thus suffers from a cardiomyopathy.

As used herein, the term **"cardiomyopathy"** has its general meaning in the art and refers to any disease of the heart muscle. Cardiomyopathy can be acquired or inherited. In some embodiments, the cardiopathy is an non-ischemic cardiomyopathy, more particularly myocardial infarction. Common symptoms include dyspnea and peripheral oedema, and risks of having dangerous forms of irregular heart rate and sudden cardiac death are increased. Cardiomyopathy often leads to progressive heart failure, i.e the incapacity of the cardiac pump to maintain sufficient blood flow to meet the basal bodily needs for oxygen. The main types of cardiomyopathy include dilated cardiomyopathy, hypertrophic cardiomyopathy, non-obstructive cardiomyopathy, restrictive cardiomyopathy, left ventricular non-compaction, arrhythmogenic right ventricular cardiomyopathy. Dilated cardiomyopathy is characterized by dilatation and systolic dysfunction of the left or both ventricles. The ventricular walls become thin and stretched, compromising cardiac contractility and ultimately resulting in poor left ventricular function.

In some embodiments, the cardiomyopathy may derive from the following non familial causes: obesity, infants of diabetic mothers, athletic training, amyloid (al/prealbumin), myocarditis (infective/toxic/immune), Kawasaki disease, eosinophilic (churg strauss, syndrome), viral persistence, pregnancy, endocrine, nutritional (thiamine, carnitine, selenium, hypophosphataemia, hypocalcaemia), alcohol, tachycardiomyopathy, inflammation, amyloid (AL/prealbumin), scleroderma, endomyocardial fibrosis, hypereosinophilic syndrome, drugs (serotonin, methysergide,, ergotamine, mercurial agents, busulfan), carcinoid heart disease, metastatic cancers, antineoplastic drugs (anthracyclines, antimetabolites; alkylating agents; taxol, hypomethylating agent, monoclonal antibodies, tyrosine kinase inhibitors, immunomodulating agents), psychiatric drugs (clozapine, olanzapine; chlorpromazine, risperidone, lithium, methylphenidate, tricyclic antidepressants) and other drugs such as chloroquine, all-trans retinoic acid, antiretro viral agents and phenothiazines. More particularly, the cardiomyopathy results from direct and indirect sympathomimetics, beta-blockers, anticholinergics, cholinomimetics, adenosine receptor agonist, Ca²⁺ channel blockers, Na⁺/^{K+-}ATPase inhibitors, lysosomotropic agents, anticancer chemotherapeutics (such as cytarabine, cyclophosphamide, daunorubicin, docetaxel, epirubicin, paclitaxel, treosulfan and other anthracyclines, alkylating agents and taxanes), HER2-targeting antibodies (such as adotrastuzumab trastuzumab, pertuzumab and others), tyrosine kinase inhibitors (such as dobrafenib, lapatinib, sorafenib, sunitinib, ponatinib and others), proteasome inhibitors (such as bortezomib, karfilzomib and others), non-steroidal anti-inflammatory agents, ethanol, nicotine and smoking.

In some embodiments, the patient suffers from a metabolic cardiomyopathy, in particular, from an age-related metabolic cardiomyopathy.

As used herein, the term **"metabolic cardiomyopathy"** relates to a cardiomyopathy associated with metabolic syndrome, such as obesity, diabetes/insulin resistance, hypertension and dyslipidemia. In particular, **"metabolic cardiomyopathy** " refers to cardiac consequences of metabolic syndrome such as atherosclerosis, coronary heart disease, obesity-associated heart disease, insulin resistance- associated heart disease, hypertensive heart disease, cardiac remodeling, heart failure and cardiometabolic diseases disclosed in Hertle et al, 2014; Hua and Nair, 2014; U.S. Pat. Application No. 2012/0214771 and International Patent Application No. 2008/094939. As used herein, the term **"age related metabolic cardiomyopathy"** relates to any metabolic cardiomyopathy which has a factor of its etiology the age of the patient.

In some embodiments, the patient suffers from heart failure, in particular heart failure with preserved ejection fraction.

As used herein, the term **"heart failure"** or **"HF"** has its general meaning in the art and embraces congestive heart failure and/or chronic heart failure. Functional classification of heart failure is generally done by the New York Heart Association Functional Classification (Criteria Committee, New York Heart Association. Diseases of the heart and blood vessels. Nomenclature and criteria for diagnosis, 6th ed. Boston: Little, Brown and co, 1964;114). This classification stages the severity of heart failure into 4 classes (I-IV). The classes (I-IV) are: Class I: no limitation is experienced in any activities; there are no symptoms from ordinary activities; Class II: slight, mild limitation of activity; the patient is comfortable at rest or with mild exertion; Class III: marked limitation of any activity; the patient is comfortable only at rest; Class IV: any physical activity brings on discomfort and symptoms occur at rest.

As used herein, the term **"heart failure with preserved ejection fraction"** has its general meaning in the art and refers to a complex syndrome characterized by heart failure (HF) signs and symptoms and a normal or near-normal left ventricular ejection fraction (LVEF). More specific diagnostic criteria include signs/symptoms of HF, objective evidence of diastolic dysfunction, disturbed left ventricular (LV) filling, structural heart disease, and elevated brain natriuretic peptides. Additional cardiac abnormalities can include subtle alterations of systolic function, impaired atrial function, chronotropic incompetence, or haemodynamic alterations, such as elevated pre-load volumes.

As used herein, the term **"DBI"** has its general meaning in the art and refers to the diazepam binding inhibitor, acyl-CoA binding protein encoding by the *DBI gene* (Gene ID: 1622). The term is also known as EP; ACBP; ACBD1; and CCK-RP. An exemplary amino acid sequence for DBI is represented by SEQ ID NO:1.

SEQ ID NO:1 >sp|P07108|ACBP_HUMAN Acyl-CoA-binding protein OS=Homo
sapiens OX=9606 GN=DBI PE=1 SV=2
MSQAEFEKAAEEVRHLKTKPSDEEMLFIYGHYKQATVGDINTERPGMLDFTGKAKWDAWN
ELKGTSKEDAMKAYINKVEELKKKYGI

In some embodiments, the agent that inhibits the activity of DBI is an antibody directed against DBI. In some embodiment, the agent is thus a neutralizing anti-DBI monoclonal antibody.

As used herein the term **"antibody"** and **"immunoglobulin"** have the same meaning, and will be used equally in the present invention. The term **"antibody"** as used herein refers to immunoglobulin molecules and immunologically active portions of immunoglobulin molecules, i.e., molecules that contain an antigen binding site that immunospecifically binds an antigen. As such, the term antibody encompasses not only whole antibody molecules, but also antibody fragments as well as variants (including derivatives) of antibodies and antibody fragments. In natural antibodies, two heavy chains are linked to each other by disulfide bonds and each heavy chain is linked to a light chain by a disulfide bond. There are two types of light chain, lambda (1) and kappa (k). There are five main heavy chain classes (or isotypes) which determine the functional activity of an antibody molecule: IgM, IgD, IgG, IgA and IgE. Each chain contains distinct sequence domains. The light chain includes two domains, a variable domain (VL) and a constant domain (CL). The heavy chain includes three (α, δ, γ) to five (µ, ε) domains, a variable domain (VH) and three to four constant domains (CH1, CH2, CH3 and CH4 collectively referred to as CH). The variable regions of both light (VL) and heavy (VH) chains determine binding recognition and specificity to the antigen. The constant region domains of the light (CL) and heavy (CH) chains confer important biological properties such as antibody chain association, secretion, trans-placental mobility, complement binding, and binding to Fc receptors (FcR). The Fv fragment is the N-terminal part of the Fab fragment of an immunoglobulin and consists of the variable portions of one light chain and one heavy chain. The specificity of the antibody resides in the structural complementarity between the antibody combining site and the antigenic determinant. Antibody combining sites are made up of residues that are primarily from the hypervariable or complementarity determining regions (CDRs). Occasionally, residues from nonhypervariable or framework regions (FR) can participate to the antibody binding site or influence the overall domain structure and hence the combining site. CDRs refer to amino acid sequences which together define the binding affinity and specificity of the natural Fv region of a native immunoglobulin binding site. The light and heavy chains of an immunoglobulin each have three CDRs, designated L-CDR1, L-CDR2, L-CDR3 and H-CDR1, H-CDR2, H-CDR3, respectively. An antigen-binding site, therefore, typically includes six CDRs, comprising the CDR set from each of a heavy and a light chain V region. Framework Regions (FRs) refer to amino acid sequences interposed between CDRs. The residues in antibody variable domains are conventionally numbered according to a system devised by Kabat et al. This system is set forth in Kabat et al., 1987, in Sequences of Proteins of Immunological Interest, US Department of Health and Human Services, NIH, USA (hereafter "Kabat et al."). This numbering system is used in the present specification. The Kabat residue designations do not always correspond directly with the linear numbering of the amino acid residues in SEQ ID sequences. The actual linear amino acid sequence may contain fewer or additional amino acids than in the strict Kabat numbering corresponding to a shortening of, or insertion into, a structural component, whether framework or complementarity determining region (CDR), of the basic variable domain structure. The correct Kabat numbering of residues may be determined for a given antibody by alignment of residues of homology in the sequence of the antibody with a "standard" Kabat numbered sequence. The CDRs of the heavy chain variable domain are located at residues 31-35B (H-CDR1), residues 50-65 (H-CDR2) and residues 95-102 (H-CDR3) according to the Kabat numbering system. The CDRs of the light chain variable domain are located at residues 24-34 (L-CDR1), residues 50-56 (L-CDR2) and residues 89-97 (L-CDR3) according to the Kabat numbering system.

As used herein, the term **"neutralizing anti-DBI monoclonal antibody"** refers to an antibody to a monoclonal antibody having specificity for DBI and that inhibits, reduces or completely the activity of DBI. Whether an antibody is a neutralizing antibody can be determined by in vitro assays described in the EXAMPLE. Typically, the neutralizing antibody of the present invention inhibits the activity of DBI by at least 50%, 60%, 70%, 80%, 90%, 95%, 99%, or 100%.

As used herein, the terms **"monoclonal antibody", "monoclonal Ab", "monoclonal antibody composition", "mAb",** or the like, as used herein refer to a preparation of antibody molecules of single molecular composition. A monoclonal antibody is obtained from a population of substantially homogeneous antibodies, *i.e.,* the individual antibodies comprised in the population are identical except for possible naturally occurring mutations that may be present in minor amounts.

In some embodiments, the antibody is directed against the fragment consisting in the amino acid sequence ranging from the amino acid residue at position 43 to the amino acid residue at position 50 in SEQ ID NO:1 (i.e. the octapeptide or OP).

In some embodiments, the antibody of the present invention is a chimeric antibody, typically a chimeric mouse/human antibody.

As used herein, the term **"chimeric antibody"** refers to an antibody which comprises a VH domain and a VL domain of a non-human antibody, and a CH domain and a CL domain of a human antibody. In some embodiments, a **"chimeric antibody"** is an antibody molecule in which (a) the constant region *(i.e.,* the heavy and/or light chain), or a portion thereof, is altered, replaced or exchanged so that the antigen binding site (variable region) is linked to a constant region of a different or altered class, effector function and/or species, or an entirely different molecule which confers new properties to the chimeric antibody, e.g., an enzyme, toxin, hormone, growth factor, drug, etc.; or (b) the variable region, or a portion thereof, is altered, replaced or exchanged with a variable region having a different or altered antigen specificity.

Chimeric antibodies also include primatized and in particular humanized antibodies. Furthermore, chimeric antibodies may comprise residues that are not found in the recipient antibody or in the donor antibody. These modifications are made to further refine antibody performance. For further details, see Jones et al., Nature 321:522-525 (1986); Riechmann et al., Nature 332:323-329 (1988); and Presta, Curr. Op. Struct. Biol. 2:593-596 (1992). (see U.S. Pat. No. 4,816,567; and Morrison et al., Proc. Natl. Acad. Sci. USA, 81:6851-6855 (1984)).

In some embodiments, the antibody is a humanized antibody.

As used hereon, the term **"humanized antibody"** refers to an antibody having variable region framework and constant regions from a human antibody but retains the CDRs of a previous non-human antibody. In some embodiments, a humanized antibody contains minimal sequence derived from non-human immunoglobulin. For the most part, humanized antibodies and antibody fragments thereof may be human immunoglobulins (recipient antibody or antibody fragment) in which residues from a complementary-determining region (CDR) of the recipient are replaced by residues from a CDR of a non-human species (donor antibody) such as mouse, rat or rabbit having the desired specificity, affinity, and capacity. In some instances, Fv framework region (FR) residues of the human immunoglobulin are replaced by corresponding non-human residues. Furthermore, a humanized antibody/antibody fragment can comprise residues which are found neither in the recipient antibody nor in the imported CDR or framework sequences. Such antibodies are designed to maintain the binding specificity of the non-human antibody from which the binding regions are derived, but to avoid an immune reaction against the non-human antibody. These modifications can further refine and optimize antibody or antibody fragment performance. In general, the humanized antibody or antibody fragment thereof will comprise substantially all of at least one, and typically two, variable domains, in which all or substantially all of the CDR regions correspond to those of a non-human immunoglobulin and all or a significant portion of the FR regions are those of a human immunoglobulin sequence. The humanized antibody or antibody fragment can also comprise at least a portion of an immunoglobulin constant region (Fc), typically that of a human immunoglobulin. For further details, see Jones et al., Nature, 321: 522-525, 1986; Reichmann et al., Nature, 332: 323-329, 1988; Presta, Curr. Op. Struct. Biol., 2: 593-596, 1992.

In some embodiments, the antibody is a human antibody.

As used herein the term **"human antibody"** as used herein, is intended to include antibodies having variable and constant regions derived from human immunoglobulin sequences. The human antibodies of the present invention may include amino acid residues not encoded by human immunoglobulin sequences (e.g., mutations introduced by random or site-specific mutagenesis in vitro or by somatic mutation in vivo). However, the term **"human antibody",** as used herein, is not intended to include antibodies in which CDR sequences derived from the germline of another mammalian species, such as a mouse, have been grafted onto human framework sequences.

In some embodiments, the neutralizing antibody of the present invention does not mediate antibody-dependent cell-mediated cytotoxicity and thus does not comprise an Fc portion that induces antibody dependent cellular cytotoxicity (ADCC). In some embodiments, the neutralizing antibody does not comprise an Fc domain capable of substantially binding to a FcgRIIIA (CD16) polypeptide. In some embodiments, the neutralizing antibody lacks an Fc domain (e.g. lacks a CH2 and/or CH3 domain) or comprises an Fc domain of IgG2 or IgG4 isotype. In some embodiments, the neutralizing antibody consists of or comprises a Fab, Fab', Fab'-SH, F (ab') 2, Fv, a diabody, single-chain antibody fragment, or a multispecific antibody comprising multiple different antibody fragments. In some embodiments, the neutralizing antibody is not linked to a toxic moiety. In some embodiments, one or more amino acids selected from amino acid residues can be replaced with a different amino acid residue such that the antibody has altered C2q binding and/or reduced or abolished complement dependent cytotoxicity (CDC). This approach is described in further detail in U.S. Patent Nos. 6,194,551 by ldusogie et al.

In some embodiments, the agent that inhibits the activity of DBI is an aptamer directed against DBI. Aptamers are a class of molecule that represents an alternative to antibodies in term of molecular recognition. Aptamers are oligonucleotide sequences with the capacity to recognize virtually any class of target molecules with high affinity and specificity. Such ligands may be isolated through Systematic Evolution of Ligands by EXponential enrichment (SELEX) of a random sequence library. The random sequence library is obtainable by combinatorial chemical synthesis of DNA. In this library, each member is a linear oligomer, eventually chemically modified, of a unique sequence. Peptide aptamers consists of a conformationally constrained antibody variable region displayed by a platform protein, such as E. coli Thioredoxin A that are selected from combinatorial libraries by two hybrid methods (Colas et al., 1996).

In some embodiments, the agent that inhibits the expression of DBI is an inhibitor of expression. In a preferred embodiment of the invention, said inhibitor of gene expression is a siRNA, an endonuclease, an antisense oligonucleotide or a ribozyme.

In some embodiments, the agent that inhibits the activity of DBI consists in a vaccine composition suitable for eliciting neutralizing autoantibodies against DBI when administered to the subject. For the purpose of the present invention, the term "vaccine composition" is intended to mean a composition which can be administered to humans or to animals in order to induce an immune system response; this immune system response can result in the production of antibodies against DBI. Typically, the vaccine composition comprises at least one antigen derived from DBI. As used herein the term **"antigen"** refers to a molecule capable of being specifically bound by an antibody or by a T cell receptor (TCR) if processed and presented by MHC molecules. The term "antigen", as used herein, also encompasses T-cell epitopes. An antigen is additionally capable of being recognized by the immune system and/or being capable of inducing a humoral immune response and/or cellular immune response leading to the activation of B- and/or T-lymphocytes. An antigen can have one or more epitopes or antigenic sites (B- and T- epitopes). In some embodiments, the antigen consists in a polypeptide comprising an amino acid sequence having at least 80% of identity with the sequence of SEQ ID NO:1 or a fragment thereof (e.g. an epitope). In some embodiments, the antigen consists in a polypeptide comprising i) an amino acid sequence having at least 80% of identity with SEQ ID NO:1, or ii) an amino acid sequence having at least 80% of identity with the amino acid sequence ranging from the amino acid residue at position 17 to the amino acid residue at position 50 in SEQ ID NO:1, or iii) an amino acid sequence having at least 80% of identity with the amino acid sequence ranging from the amino acid residue at position 33 to the amino acid residue at position 50 in SEQ ID NO:1, or iv) an amino acid sequence having at least 80% of identity with the amino acid sequence ranging from the amino acid residue at position 43 to the amino acid residue at position 50 in SEQ ID NO:1. In some embodiments, the polypeptide is conjugated to a carrier protein which is generally sufficiently foreign to elicit a strong immune response to the vaccine. Illustrative carrier proteins are inherently highly immunogenic. Both bovine serum albumin (BSA) and keyhole limpet hemocyanin (KLH) have commonly been used as carriers in the development of conjugate vaccines when experimenting with animals and are contemplated herein as carrier proteins. Proteins which have been used in the preparation of therapeutic conjugate vaccines include, but are not limited to, a number of toxins of pathogenic bacteria and their toxoids. Suitable carrier molecules are numerous and include, but are not limited to: Bacterial toxins or products, for example, cholera toxin B-(CTB), diphtheria toxin, tetanus toxoid, and pertussis toxin and filamentous hemagglutinin, shiga toxin, pseudomonas exotoxin; Lectins, for example, ricin-B subunit, abrin and sweet pea lectin; Sub virals, for example, retrovirus nucleoprotein (retro NP), rabies ribonucleoprotein (rabies RNP), plant viruses (e.g. TMV, cow pea and cauliflower mosaic viruses), vesicular stomatitis virus-nucleocapsid protein (VSV-N), poxvirus vectors and Semliki forest virus vectors; Artificial vehicles, for example, multiantigenic peptides (MAP), microspheres; Yeast virus-like particles (VLPs); Malarial protein antigen; and others such as proteins and peptides as well as any modifications, derivatives or analogs of the above. Other useful carriers include those with the ability to enhance a mucosal response, more particularly, LTB family of bacterial toxins, retrovirus nucleoprotein (retro NP), rabies ribonucleoprotein (rabies RNP), vesicular stomatitis virus-nucleocapsid protein (VSV-N), and recombinant .pox virus subunits.

As used herein, the term **"therapeutically effective amount"** refers to an amount effective, at dosages and for periods of time necessary, to achieve a desired therapeutic result. A therapeutically effective amount of the active agent may vary according to factors such as the disease state, age, sex, and weight of the individual, and the ability of the active agent to elicit a desired response in the individual. A therapeutically effective amount is also one in which any toxic or detrimental effects of drug are outweighed by the therapeutically beneficial effects. The efficient dosages and dosage regimens for the active agent depend on the disease or condition to be treated and may be determined by the persons skilled in the art. A physician having ordinary skill in the art may readily determine and prescribe the effective amount of the pharmaceutical composition required. For example, the physician could start doses of active agent employed in the pharmaceutical composition at levels lower than that required achieving the desired therapeutic effect and gradually increasing the dosage until the desired effect is achieved. In general, a suitable dose of a composition of the present invention will be that amount of the compound, which is the lowest dose effective to produce a therapeutic effect according to a particular dosage regimen. Such an effective dose will generally depend upon the factors described above. For example, a therapeutically effective amount for therapeutic use may be measured by its ability to stabilize the progression of disease. One of ordinary skill in the art would be able to determine such amounts based on such factors as the patient's size, the severity of the patient's symptoms, and the particular composition or route of administration selected. An exemplary, non-limiting range for a therapeutically effective amount of a drug of the present invention is about 0.1-100 mg/kg, such as about 0.1-50 mg/kg, for example about 0.1-20 mg/kg, such as about 0.1-10 mg/kg, for instance about 0.5, about such as 0.3, about 1, about 3 mg/kg, about 5 mg/kg or about 8 mg/kg. An exemplary, non-limiting range for a therapeutically effective amount of a drug of the present invention is 0.02-100 mg/kg, such as about 0.02-30 mg/kg, such as about 0.05-10 mg/kg or 0.1-3 mg/kg, for example about 0.5-2 mg/kg.

Typically, the agent that inhibits the activity or expression of DBI is administered to the patient in the form of a pharmaceutical composition which comprises a pharmaceutically acceptable carrier.

As used herein, the term **"pharmaceutical composition"** refers to a composition described herein, or pharmaceutically acceptable salts thereof, with other agents such as carriers and/or excipients. The pharmaceutical compositions as provided herewith typically include a pharmaceutically acceptable carrier.

As used herein, the term **"pharmaceutically acceptable carrier"** includes any and all solvents, diluents, or other liquid vehicle, dispersion or suspension aids, surface active agents, isotonic agents, thickening or emulsifying agents, preservatives, solid binders, lubricants and the like, as suited to the particular dosage form desired. Remington's Pharmaceutical-Sciences, Sixteenth Edition, E. W. Martin (Mack Publishing Co., Easton, Pa., 1980) discloses various carriers used in formulating pharmaceutical compositions and known techniques for the preparation thereof.

Pharmaceutically acceptable carriers that may be used in these compositions include, but are not limited to, ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene- block polymers, polyethylene glycol and wool fat. For use in administration to a patient, the composition will be formulated for administration to the patient. The compositions of the present invention may be administered orally, parenterally, by inhalation spray, topically, rectally, nasally, buccally, vaginally or via an implanted reservoir. The used herein includes subcutaneous, intravenous, intramuscular, intra-articular, intra-synovial, intrasternal, intrathecal, intrahepatic, intralesional and intracranial injection or infusion techniques. Sterile injectable forms of the compositions of this invention may be aqueous or an oleaginous suspension. These suspensions may be formulated according to techniques known in the art using suitable dispersing or wetting agents and suspending agents. The sterile injectable preparation may also be a sterile injectable solution or suspension in a non-toxic parenterally acceptable diluent or solvent, for example as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium. For this purpose, any bland fixed oil may be employed including synthetic mono-or diglycerides. Fatty acids, such as oleic acid and its glyceride derivatives are useful in the preparation of injectables, as are natural pharmaceutically-acceptable oils, such as olive oil or castor oil, especially in their polyoxyethylated versions. These oil solutions or suspensions may also contain a long-chain alcohol diluent or dispersant, such as carboxymethyl cellulose or similar dispersing agents that are commonly used in the formulation of pharmaceutically acceptable dosage forms including emulsions and suspensions. Other commonly used surfactants, such as Tweens, Spans and other emulsifying agents or bioavailability enhancers which are commonly used in the manufacture of pharmaceutically acceptable solid, liquid, or other dosage forms may also be used for the purposes of formulation. The compositions of this invention may be orally administered in any orally acceptable dosage form including, but not limited to, capsules, tablets, aqueous suspensions or solutions. In the case of tablets for oral use, carriers commonly used include lactose and corn starch. Lubricating agents, such as magnesium stearate, are also typically added. For oral administration in a capsule form, useful diluents include, e.g., lactose. When aqueous suspensions are required for oral use, the agent that inhibits the activity or expression of DBI is combined with emulsifying and suspending agents. If desired, certain sweetening, flavoring or coloring agents may also be added. Alternatively, the compositions of this invention may be administered in the form of suppositories for rectal administration. These can be prepared by mixing the agent with a suitable non-irritating excipient that is solid at room temperature but liquid at rectal temperature and therefore will melt in the rectum to release the drug. Such materials include cocoa butter, beeswax and polyethylene glycols. The compositions of this invention may also be administered topically, especially when the target of treatment includes areas or organs readily accessible by topical application, including diseases of the eye, the skin, or the lower intestinal tract. Suitable topical formulations are readily prepared for each of these areas or organs. For topical applications, the compositions may be formulated in a suitable ointment containing the active component suspended or dissolved in one or more carriers. Carriers for topical administration of the compounds of this invention include, but are not limited to, mineral oil, liquid petrolatum, white petrolatum, propylene glycol, polyoxyethylene, polyoxypropylene compound, emulsifying wax and water. Alternatively, the compositions can be formulated in a suitable lotion or cream containing the active components suspended or dissolved in one or more pharmaceutically acceptable carriers. Suitable carriers include, but are not limited to, mineral oil, sorbitan monostearate, polysorbate 60, cetyl esters wax, cetearyl alcohol, 2-octyldodecanol, benzyl alcohol and water. Topical application for the lower intestinal tract can be effected in a rectal suppository formulation (see above) or in a suitable enema formulation. Patches may also be used. The compositions of this invention may also be administered by nasal aerosol or inhalation. Such compositions are prepared according to techniques well-known in the art of pharmaceutical formulation and may be prepared as solutions in saline, employing benzyl alcohol or other suitable preservatives, absorption promoters to enhance bioavailability, fluorocarbons, and/or other conventional solubilizing or dispersing agents.

The invention will be further illustrated by the following figures and examples. However, these examples and figures should not be interpreted in any way as limiting the scope of the present invention.

### FIGURES:

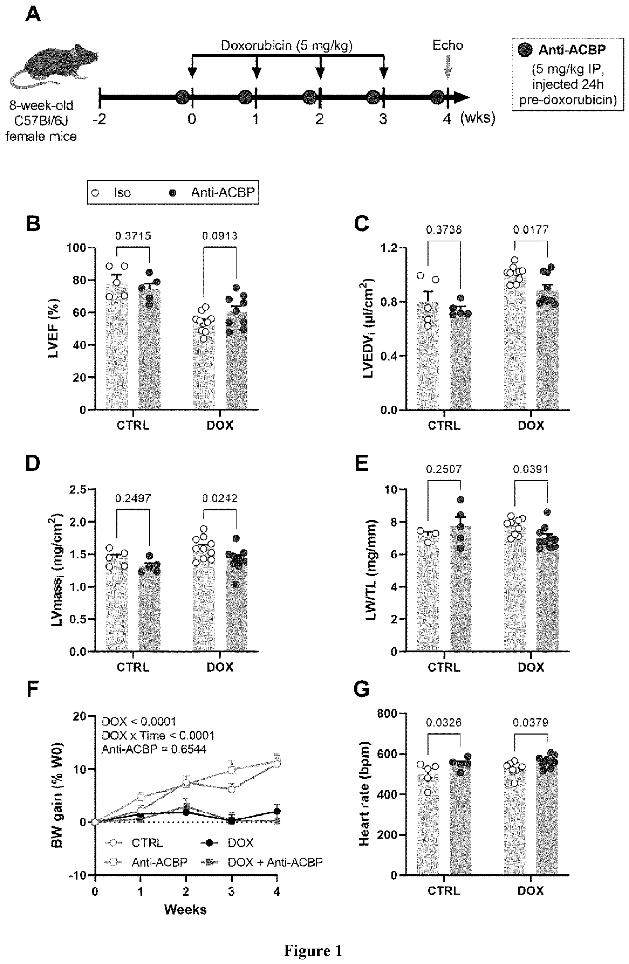

**Figure 1****. ACBP neutralization attenuates anthracyline-induced cardiotoxicity**
   Schematic representation of ACBP-neutralizing antibody (anti-ACBP) administration to doxorubicin (DOX)-treated C57B1/6J female mice.
   A. Echocardiography-derived left ventricular ejection fraction (LVEF).
   B. Left ventricular end-diastolic volume normalized to body surface area (LVEDVᵢ)
   C. Left ventricular mass index (LVmassᵢ), calculated as the ratio between LVmass and body surface area.
   D. Tibia length-normalized lung weight (LW/TL)
   E. Body weight (BW) gain
   F. Heart rate
   N=3-10 mice per groups. P values on top of the panels (B-E and G) represent pairwise comparisons between anti-ACBP-treated mice and their respective isotype (Iso)-treated controls using simple main effects of a factorial ANOVA including DOX and anti-ACBP as fixed factors. In (F), P values were calculated using a mixed model, including DOX, anti-ACBP and Time as fixed factors. Bars and error bars show means and SEM, respectively, with individual data points superimposed. Abbreviations: bpm, beat per minute; echo, echocardiography.

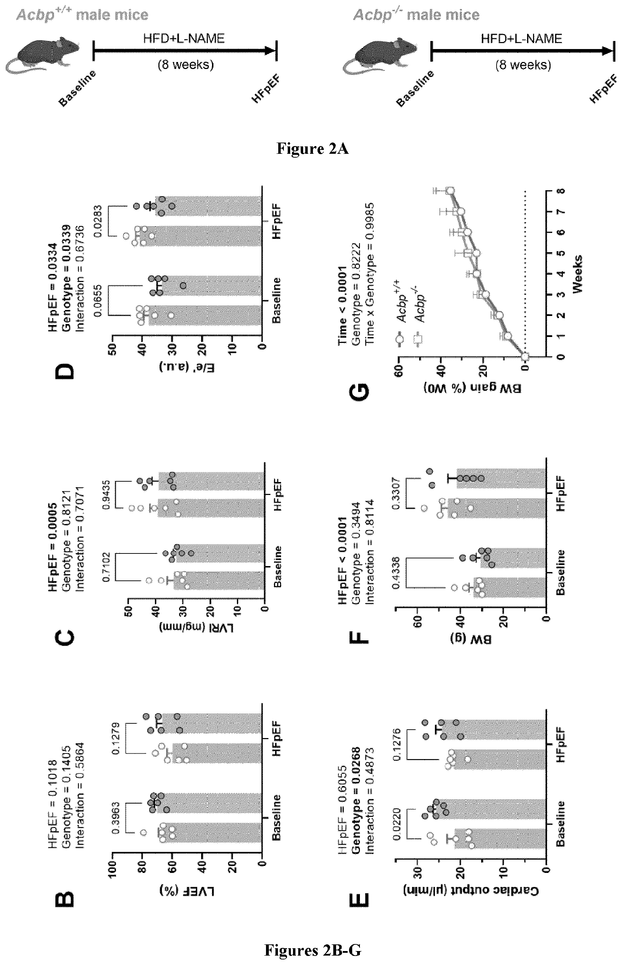

**Figure 2****. Cardioprotection against HFpEF in ACBP knockout mice**
   A. High-fat diet (HFD) and N[ω]-nitro-1-arginine methyl ester (L-NAME) were administered to Acbp-expressing (Acbp+/+) and Acbp-deficient (Acbp-/-) male mice for 8 weeks, with the following echocardiography-derived parameters determined at week 0 (baseline) and week 8 (HFpEF):
   B. Left ventricular ejection fraction (LVEF).
   C. Left ventricular remodelling index (LVRI), calculated as the ratio of LV mass to internal diastolic diameter
   D. Ratio of peak early Doppler transmitral flow velocity (E) to myocardial tissue Doppler velocity (e'), a measure of diastolic dysfunction.
   E. Cardiac output
   F. Body weight (BW)
   G. Body weight (BW) gain
   N=6 mice per groups. P values on top of the panels represent factor comparisons by 2-way repeated-measures ANOVA including Genotype and the HFpEF protocol (B-F) or Genotype and Time (G) as fixed factors, followed by simple main effects analysis of pairwise comparisons between Acbp+/+ and Acbp-/- mice. Bars and error bars show means and SEM, respectively, with individual data points superimposed.

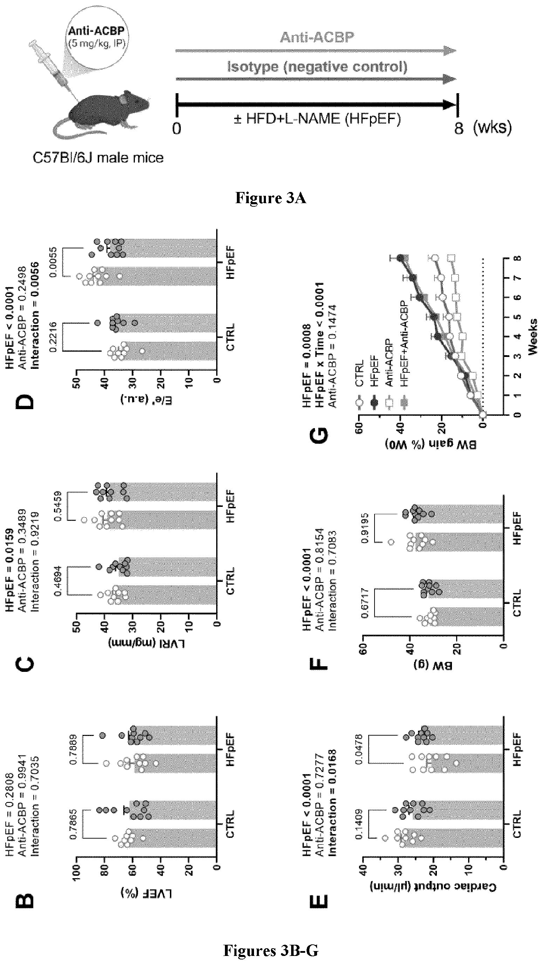

**Figure 3****. Cardioprotection against HFpEF using an ACBP-neutralizing antibody**
   A. High-fat diet (HFD) and N[ω]-nitro-1-arginine methyl ester (L-NAME) were administered to C57B1/6J male mice (10-week-old), which were either treated with an ACBP-neutralizing antibody (anti-ACBP) or mouse isotype IgG (CTRL) for 8 weeks. Subsequently, the following echocardiography-derived parameters were determined:
   B. Left ventricular ejection fraction (LVEF)
   C. Left ventricular remodelling index (LVRI), calculated as the ratio of LV mass to internal diastolic diameter
   D. Ratio of peak early Doppler transmitral flow velocity (E) to myocardial tissue Doppler velocity (e').
   E. Cardiac output
   F. Body weight (BW)
   G. Body weight (BW) gain
   N=9-10 mice per groups. P values on top of the panels (B-F) represent factor comparisons by a 2-way independent ANOVA including anti-ACBP treatment and the two-hit HFpEF model as fixed factors, followed by simple main effects analysis of pairwise comparisons between Anti-ACBP-treated mice and their respective isotype-treated controls. In (G), P values were calculated using a mixed model, including HFpEF, Anti-ACBP and Time as fixed factors. Bars and error bars show means and SEM, respectively, with individual data points superimposed.

### EXAMPLE:

### Introduction:

Acyl coenzyme A binding protein (ACBP), which is encoded by *diazepam-binding inhibitor* (*DBI*), is a leaderless polypeptide that can be secreted in an unconventional fashion during the activation of autophagy (1). We have found that circulating ACBP/DBI levels increase with aging and obesity (1,2). Hence, we wonder whether inhibition of ACBP/DBI might reduce cardiac disease associated with aging and disease. Therefore, we used an animal model of anthracycline-induced cardiomyocyte senescence (3,4) to determine whether ACBP is causally involved in cardiac disease. Heart failure with preserved ejection fraction (HFpEF) is currently the predominant form of heart failure and the leading cause of hospitalization in the elderly. However, limited understanding of the underlying mechanisms of HFpEF has led to a longstanding absence of evidence-based therapies. In this respect, growing epidemiological and experimental evidence suggests that metabolic dysfunction might drive the pathogenesis of HFpEF. In fact, the majority of HFpEF patients are obese or diabetic, suggesting that metabolic therapies acting on the heart and peripheral organs are worth considering (5). To this end, we hypothesized that ACBP/DBI might be therapeutically targeted for the treatment of HFpEF.

### Methods

**Cardiac toxicity experiments:** Eight-week-old C57B1/6J female mice were purchased from Envigo (Gannat, France). Following 2 weeks of acclimatization, mice were randomized (1:1) to receive either mouse monoclonal anti-ACBP-neutralizing antibody (5 mg/kg body weight, injected intraperitoneally once per week) or mouse isotype IgG (negative control; 5 mg/kg body weight). Each group was further randomized (1:1) to receive a weekly intraperitoneal injection of saline or doxorubicin (5 mg/kg body weight; cumulative dose of 20 mg/kg body weight over 4 weeks) (Sigma, #MKCM8540) (7). Of note, anti-ACBP was always administered 24 hours prior to doxorubicin injection. All mice used in this study were housed in a temperature-controlled environment with 12 h light/dark cycles and *ad libitum* access to food and water.

**HFpEF model:** Eight-week-old C57B1/6J male mice were purchased from Envigo (Gannat, France). Following 2 weeks of acclimatization, mice were randomized (1:1) to receive either HFD (Safe, #260 HF) and L-NAME (0.5 g/l in the drinking water) or a regular diet (Safe, #A04) and normal drinking water. Each group was further randomized (1:1) to receive mouse monoclonal anti-ACBP-neutralizing antibody (5 mg/kg body weight, injected intraperitoneally once per week) or mouse isotype IgG (negative control; 5 mg/kg body weight). As for conditional deletion of Acbp, we generated *Acbp*^{*-*/*-*} mice by crossing B6.Cg-Tg(UBC-cre/ERT2)1Ejb/1J mice (Jackson Laboratory, Bar Harbor, ME, USA) and *Acbp*^{*fl*/*fl*} mice (OZgene, Bentley, WA, USA). Genotype was verified by PCR, as described (8) At the age of 8 weeks, Cre recombinase was activated by tamoxifen injections (75 mg/kg body weight IP, daily for 5 days). HFD+L-NAME treatment was initiated in *Acbp*^{*-*/*-*} and their *Acbp*^{+/+} littermates 8 weeks after the last tamoxifen injection, thereby avoiding transient tamoxifen-related cardiomyopathy (9). After 8 weeks of HFD+L-NAME treatment, all mice were assessed using non-invasive echocardiography (Vevo3100, Fujifilm VisualSonics Inc., Canada), as described (10). Animal experiments were performed according to the European ethical regulations (Directive 2010/63/EU) and were approved by the Animal Experimental Ethics Committee in France (protocol #35132).

**Echocardiography.** Mice were lightly anesthetized (5% for induction; 1.5% isoflurane for maintenance) and body temperature was kept at 37°C using a temperature-controlled heating platform. Mice were placed in a supine position with their limbs in direct contact with non-invasive electrocardiogram leads for heart rate assessment. Pre-warmed ultrasound transmission gel was spread on a shaved chest to obtain cardiac tracings in the parasternal long axis using high-resolution 55 MHz linear-array probe. M-mode tracings were used to evaluate cardiac walls thickness and internal left ventricular dimensions at the level of the papillary muscles during systole and diastole. Ventricular volumes and myocardial mass were estimated using Teichholtz and Troy formulas, respectively. Ejection fraction was determined to assess systolic function, while diastolic function was evaluate using the ratio between peak early-filling velocity of transmitral flow (E) and the corresponding mitral valve annulus velocity (e') determined using pulsed-wave and tissue Doppler imaging, respectively. Generally, at least 3 stable cardiac cycles were averaged to obtain the reported parameters.

### Results:

**ACBP/DBI neutralization reduces anthracycline-accelerated cardiac aging.** We used an animal model of anthracycline-induced cardiomyocyte senescence (3,4) to determine whether ACBP is causally involved in cardiac disease (Fig. 1A). Mice were subjected to chronic doxorubicin (DOX) treatment (cumulative dose: 20 mg/kg body weight, injected intraperitoneally over 4 weeks). DOX treatment clearly reduced left ventricular ejection fraction (p<0:001, Fig. 1B), thereby causing ventricular dilation (P=0.001; Fig. 1C). By contrast, treatment of DOX mice with a murine monoclonal ACBP-neutralizing antibody (Anti-ACBP; 5 mg/kg body weight, injected IP weekly) partially preserved cardiac function, as suggested by a significant reduction in left ventricular dilation, despite an unaltered ejection fraction (Fig. 1B-C). Anti-ACBP-treated mice also exhibited lower left ventricular mass index and tibia length-normalized lung weight (Fig. 1D-E), indicating reduced cardiac remodelling and lung congestion, respectively. Of note, doxorubicin-induced suppression of body weight gain was not affected by anti-ACBP (Fig. IF), whereas anti-ACBP appeared to increase heart rate, irrespective of DOX treatment (Fig. 1G). Taken together, ACBP neutralization reduces the cardiotoxicity of elevated anthracycline doses.

### ACBP/DBI neutralization reduces HFpEF.

To examine whether ACBP neutralization improves HFpEF in mice, we generated Acbp knockout mice (*Acbp*^{*-*/*-*}), in which Acbp was conditionally depleted upon tamoxifen administration. Male *Acbp*^{*-*/*-*} and their wild type littermates (*Acbp*^{+/+}) were subjected to a 'two-hit' HFpEF model using high-fat diet (HFD) and the nitric oxide synthase inhibitor N[ω]-nitro-1-arginine methyl ester (L-NAME) (Fig. 2A) (6). After 8 weeks, cardiac structure and function were comprehensively assessed using echocardiography. HFD+L-NAME feeding did not alter ejection fraction (Fig. 2B), but effectively induced cardiac remodelling and diastolic dysfunction, as indicated by a higher left ventricular remodelling index (LVRI) and an increased E/e' (peak early Doppler transmitral flow velocity-to-myocardial tissue Doppler velocity) ratio (Fig. 2C-D). Although ejection fraction and LVRI were similar in *Acbp*^{+/+} and *Acbp*^{*-*/*-*} mice (Fig. 2B-C), *Acbp*^{*-*/*-*} mice exhibited significantly improved E/e' ratio (Fig. 2D), denoting improved diastolic function - the cardinal sign of HFpEF. ACBP-depleted mice also showed superior cardiac output as compared to their ACBP-expressing littermates (Fig. 2E), which occurred in absence of body weight alterations (Fig. 2F-G), thus, excluding potential secondary cardiac effects to reduced adiposity.

In an effort to improve the translational potential of these findings, we examined the anti-HFpEF effects of ACBP neutralization using a murine monoclonal ACBP-neutralizing antibody (anti-ACBP). For this, we subjected wild type C67B1/6J male mice to the same protocol using HFD and L-NAME with a subset of mice receiving anti-ACBP (5 mg/kg body weight, injected IP) once a week (Fig. 3A). In line *with Acbp* knockout, anti-ACBP did not alter EF or LVRI, but remarkably improved diastolic dysfunction and cardiac output without affecting relative or absolute body weight gain (Fig. 3B-G).

### Conclusions:

Here, we show that neutralization of ACBP/DBI reduces the deleterious effect of the anthracycline doxorubicin on cardiac function. Hence inhibition of ACBP/DBI or of its receptor may be useful for the prevention or treatment of anthracycline-induced heart failure or for antagonizing the cardiotoxic effects of other pharmacological agents including but not limited to direct and indirect sympathomimetics, beta-blockers, anticholinergics, cholinomimetics, adenosine receptor agonist, Ca²⁺ channel blockers, Na⁺/^{K+-}ATPase inhibitors, lysosomotropic agents, anticancer chemotherapeutics (such as cytarabine, cyclophosphamide, daunorubicin, docetaxel, epirubicin, paclitaxel, treosulfan and other anthracyclines, alkylating agents and taxanes), HER2-targeting antibodies (such as adotrastuzumab trastuzumab, pertuzumab and others), tyrosine kinase inhibitors (such as dobrafenib, lapatinib, sorafenib, sunitinib, ponatinib and others), proteasome inhibitors (such as bortezomib, karfilzomib and others), non-steroidal anti-inflammatory agents, ethanol, nicotine and smoking. Since anthracycline-induced heart failure is a model of accelerated cardiac aging, it is also plausible to use ACBP/DBI inhibition or neutralization as a method to prevent or treat aging of the cardiovascular system.

We also found that genetic and pharmacological interventions to deplete ACBP efficiently improve cardiac dysfunction associated with experimental HFpEF, a condition associated with aging, hypertension and obesity.

### References:

1. Bravo-San Pedro JM, Sica V, Martins I, Pol J, Loos F, Maiuri MC, et al. Acyl-CoA-Binding Protein Is a Lipogenic Factor that Triggers Food Intake and Obesity. Cell Metab 2019;30(4):754-67 e9 doi 10.1016/j.cmet.2019.07.010.
2. Joseph A, Chen H, Anagnostopoulos G, Montegut L, Lafarge A, Motino O, et al. Effects of acyl-coenzyme A binding protein (ACBP)/diazepam-binding inhibitor (DBI) on body mass index. Cell Death Dis 2021;12(6):599 doi 10.1038/s41419-021-03864-9.
3. Maejima Y, Adachi S, Ito H, Hirao K, Isobe M. Induction of premature senescence in cardiomyocytes by doxorubicin as a novel mechanism of myocardial damage. Aging Cell 2008;7(2):125-36 doi 10.1111/j.1474-9726.2007.00358.x.
4. Piegari E, De Angelis A, Cappetta D, Russo R, Esposito G, Costantino S, et al. Doxorubicin induces senescence and impairs function of human cardiac progenitor cells. Basic Res Cardiol 2013;108(2):334 doi 10.1007/s00395-013-0334-4.
5. Sedej S, Abdellatif M. Metabolic therapy for managing heart failure with preserved ejection fraction. J Mol Cell Cardiol 2022;168:68-9 doi 10.1016/j.yjmcc.2022.04.009.
6. Schiattarella GG, Altamirano F, Tong D, French KM, Villalobos E, Kim SY, et al. Nitrosative stress drives heart failure with preserved ejection fraction. Nature 2019;568(7752):351-6 doi 10.1038/s41586-019-1100-z.
7. Li M, Sala V, De Santis MC, Cimino J, Cappello P, Pianca N, et al. Phosphoinositide 3-Kinase Gamma Inhibition Protects From Anthracycline Cardiotoxicity and Reduces Tumor Growth. Circulation 2018;138(7):696-711 doi 10.1161/CIRCULATIONAHA.117.030352.
8. Abdellatif M, Leite S, Alaa M, Oliveira-Pinto J, Tavares-Silva M, Fontoura D, et al. Spectral transfer function analysis of respiratory hemodynamic fluctuations predicts end-diastolic stiffness in preserved ejection fraction heart failure. Am J Physiol Heart Circ Physiol 2016;310(1):H4-13 doi 10.1152/ajpheart.00399.2015.
9. Koitabashi N, Bedja D, Zaiman AL, Pinto YM, Zhang M, Gabrielson KL, et al. Avoidance of transient cardiomyopathy in cardiomyocyte-targeted tamoxifen-induced MerCreMer gene deletion models. Circ Res 2009;105(1):12-5 doi 10.1161/CIRCRESAHA.109.198416.
10. Abdellatif M, Trummer-Herbst V, Heberle AM, Humnig A, Pendl T, Durand S, et al. Fine-Tuning Cardiac Insulin-Like Growth Factor 1 Receptor Signaling to Promote Health and Longevity. Circulation 2022;145(25):1853-66 doi 10.1161/CIRCULATIONAHA.122.059863.

### REFERENCES:

Throughout this application, various references describe the state of the art to which this invention pertains. The disclosures of these references are hereby incorporated by reference into the present disclosure.

## Claims

1. A method of treating cardiac dysfunction in patient in need thereof comprising administering a therapeutically effective amount of an agent that inhibits the activity or expression of DBI.

2. The method of claim1 wherein the agent improves cardiac remodeling and/or cardiac fibrosis and/or impairment of systolic function (left ventricular ejection fraction (LVEF) function or strain) and/or impairment of diastolic dysfunction.

3. The method of claim 1 for the treatment of cardiac dysfunction in elderly patients, and/or obese patients and/or patients who exhibit one or more risk factors for cardiac dysfunction (e.g. age, alcohol consumption, cigarette smoking, metabolic syndrome, obesity, diabetes/insulin resistance, hypertension, dyslipidaemia, live disease or chronic kidney disease).

4. The method of claim 1 wherein the patient suffers from a cardiomyopathy.

5. The method of claim 4 wherein the patient suffers from dilated cardiomyopathy, hypertrophic cardiomyopathy, non-obstructive cardiomyopathy, restrictive cardiomyopathy, left ventricular non-compaction, or arrhythmogenic right ventricular cardiomyopathy.

6. The method of claim 4 wherein the cardiomyopathy derives from the following non familial causes: obesity, infants of diabetic mothers, athletic training, amyloid (al/prealbumin), myocarditis (infective/toxic/immune), Kawasaki disease, eosinophilic (churg strauss, syndrome), viral persistence, pregnancy, endocrine, nutritional (thiamine, carnitine, selenium, hypophosphataemia, hypocalcaemia), alcohol, tachycardiomyopathy, inflammation, amyloid (AL/prealbumin), scleroderma, endomyocardial fibrosis, hypereosinophilic syndrome, drugs (serotonin, methysergide,, ergotamine, mercurial agents, busulfan), carcinoid heart disease, metastatic cancers, antineoplastic drugs (anthracyclines, antimetabolites; alkylating agents; taxol, hypomethylating agent, monoclonal antibodies, tyrosine kinase inhibitors, immunomodulating agents), psychiatric drugs (clozapine, olanzapine; chlorpromazine, risperidone, lithium, methylphenidate, tricyclic antidepressants) and other drugs such as chloroquine, all-trans retinoic acid, antiretro viral agents and phenothiazines.

7. The method of claim 1 wherein the patient suffers from a metabolic cardiomyopathy, in particular, from an age-related metabolic cardiomyopathy.

8. The method of claim 1 wherein the patient suffers from heart failure, in particular heart failure with preserved ejection fraction.

9. The method according to any one of claims 1 to 8 wherein the agent that inhibits the activity of DBI is an antibody or an aptamer directed against DBI.

10. The method of claim 9 wherein the antibody is directed against the fragment consisting in the amino acid sequence ranging from the amino acid residue at position 43 to the amino acid residue at position 50.

11. The method of claim 9 wherein the antibody is a monoclonal chimeric antibody, a monoclonal humanized antibody, or a monoclonal human antibody.

12. The method according to any one of claims 1 to 8 wherein the agent that inhibits the expression of DBI is an inhibitor of expression, such as a siRNA, an endonuclease, an antisense oligonucleotide or a ribozyme.

13. The method according to any one of claims 1 to 8 wherein the agent that inhibits the activity of DBI consists in a vaccine composition suitable for eliciting neutralizing autoantibodies against DBI when administered to the subject.

14. The method of claim 13 wherein the vaccine composition comprises an antigen consisting in a polypeptide comprising i) an amino acid sequence having at least 80% of identity with SEQ ID NO:1, or ii) an amino acid sequence having at least 80% of identity with the amino acid sequence ranging from the amino acid residue at position 17 to the amino acid residue at position 50 in SEQ ID NO:1, or iii) an amino acid sequence having at least 80% of identity with the amino acid sequence ranging from the amino acid residue at position 33 to the amino acid residue at position 50 in SEQ ID NO:1, or iv) an amino acid sequence having at least 80% of identity with the amino acid sequence ranging from the amino acid residue at position 43 to the amino acid residue at position 50 in SEQ ID NO:1.
